# EUROPEAN PATENT SPECIFICATION

(11) **EP 3 709 024 B1**
(45) Date of publication and mention of the grant of the patent: **27.12.2023**
(21) Application number: 19162199.4
(22) Date of filing: 12.03.2019
(51) Int. Cl.: G01N 35/00, G16H 10/40, G16H 40/67, G01N 33/49

(54) **APPARATUS FOR ANALYZING BIOLOGICAL SAMPLES**
VORRICHTUNG ZUR ANALYSE VON BIOLOGISCHEN PROBEN
APPAREIL D'ANALYSE D'ÉCHANTILLONS BIOLOGIQUES

(43) Date of publication of application: 16.09.2020
(73) Proprietor: Radiometer Medical ApS, 2700 Brønshøj (DK)
(72) Inventor: Schweitz, Kasper, 2700 Brønshøj (DK); Hastrup, Morten, 2700 Brønshøj (DK); Skriver, Jakob, 2700 Brønshøj (DK)
(74) Representative: Inspicos P/S

(56) References cited:
- WO-A1-2016/195896
- US-A1- 2012 149 035

## Description

### TECHNICAL FIELD

The present invention relates to aspects of an apparatus for analyzing biological samples, in the following also referred to as an Z r analyzer, and a method of operating said apparatus. In particular, the present invention relates to a body fluid analyzer, such as a blood analyzer.

### BACKGROUND

Analyzers for measuring physical parameters of analytes in biological samples, in particular in liquid samples by means of respective analyte sensors are widely used in various industries, such as food industry, environmental industry, as well as medical and clinical industry. To ensure both accurate and precise results, the performance of such analyzers and the associated sensors is continuously scrutinized. This typically includes both detailed calibration and quality control procedures using standardized reference samples including the respective analytes in well-defined compositions. The accurate and precise operation of analyzer systems is of particular importance in clinical analysis applications for analyzing physical parameters of analytes in bodily fluids, such as whole blood. In addition to the accuracy, precision, and reliability requirements, such analyzer systems for clinical applications are also subject to further critical constraints, such as a short time to obtaining a measurement result, the capability of providing the highly reliable results from very small sample volumes, as well as requirements in terms of low operating costs, low down times, ease of use, etc. These constraints are particularly relevant in blood analyzers. Blood analyzers provide measurements of various parameters for analyzing the blood of a mammal subject, e.g. for establishing and/or monitoring a biological condition of the subject. Typically, the mammal subject is a human patient. In a variety of instances it is desirable to measure e.g. the partial pressure of blood gasses in a whole blood sample of the mammal subject, concentrations of electrolytes and metabolites in the blood sample, as well as the hematocrit value of the blood sample. For example, measuring *p*CO₂, *p*O₂, *p*H, Na⁺, K⁺, Ca²⁺, Cl⁻, Mg²⁺ glucose, lactate, creatinine, urea and hemoglobin and hemoglobin-derivate values are primary clinical indications in assessing the condition of a medical patient. A number of different analyzers currently exist for making such measurements of at least some of the above parameters. Such analyzers are able to perform precise measurements in order to provide the most meaningful diagnostic information.

The ability to efficiently perform a large number of blood analyses is important for many health care facilities. For example, patients in intensive care require a sampling frequency of 15-20 per day for blood gas and clinical chemistry measurements, leading to a potentially large numbers of blood samples to be analyzed each day. Furthermore, in order to limit the number of tests which must be performed it is desirable to gather as much information as possible upon completion of each test. Furthermore, for the same reasons, it is important that the measurements and corresponding analysis results obtained from these measurements are reliable. Each measurement is therefore typically subject to a calibration and/or quality control procedure using different rinsing, calibration and/or reference liquids and the measurement chamber is thoroughly rinsed after each measurement to avoid contamination of any subsequent measurements. Moreover, proper and continuous functioning of the sensors employed to perform the various measurements on a blood sample is of critical importance.

As the sensors typically have a limited lifetime, known sample analyzers comprise a replaceable sensor module, the sensor module comprising one or more sensors. Accordingly, the operator of the sample analyzer may remove a currently used sensor module from the sample analyzer and replace it with a new sensor module, thus allowing continued use of the sample analyzer with a fresh set of sensors.

Manufacturers of sample analyzers and sensor modules thus offer replacement sensor modules. Once manufactured, replacement sensor modules are typically stored for a certain period of time before they are inserted into a sample analyzer for the first time. For example, the replacement module may be stored at the manufacturer, at various stages of the distribution channel and/or at the health care facility where the sample analyzer is operated. Different sample analyzers may require different types of sensor modules and different sensor modules may be intended for different use, e.g. for different numbers of tests, different operational lifetime, and/or have respective other operational parameters. Accordingly sensor modules for analyzers are normally produced in many different variants regarding number of tests, configuration, lifetime etc. As a result, both vendors and customers often maintain a large inventory of sensor modules so as to ensure that the right sensor is always available.

Moreover, as different health care facilities may require the sensor modules to be employed with a sample analyzer to have different operating parameters, the manufacturer and/or distribution channels and/or customers often have to keep a number of different versions of sensor modules in stock, thus increasing inventory costs and increasing the risk that some sensor modules may reach the end of their shelf life before their actual use.

There may also be a perception of waste if the customer cannot choose a sensor module variant that precisely matches the unique customer needs. For example, a facility running 450 tests per month may be forced to either buy a sensor module configured to perform 600 tests and having a lifetime of one month or to buy a sensor module configured for 300 tests. In the first case, the customer would "waste" 150 tests; in the second case, the customer may have to endure an additional sensor startup. This problem is even accelerated when there are peaks or lows in the number of samples that are analyzed by a given analyzer, as the variation from the norm would make the problem even worse.

Another problem associated with replaceable sensor modules involves possible recalls or customer alerts in connection with potential quality issues associated with individual sensor modules or batches of sensor modules. When a sensor module manufacturer realizes a quality issue with a certain portion of manufactured sensor modules it is desirable to provide a mechanism for preventing further use of these sensor modules.

US 2012/149035 A1 discloses a cartridge for automated detection of an analyte in a bodily fluid sample.

It would thus be desirable to provide a safer and more cost effective mechanism for operating sample analyzers.

### SUMMARY OF THE INVENTION

On this background, according to a first aspect, disclosed herein are embodiments of a method of operating an apparatus for analyzing biological samples, the apparatus comprising:
- a receptacle for receiving a replaceable sensor module, the sensor module comprising one or more sensors and a writable memory;
- a measurement unit configured to bring a biological sample into operational interaction with at least a first sensor of the one or more sensors of a sensor module received by said receptacle, to obtain a measurement result responsive to the interaction between at least the first sensor and the biological sample and to output the obtained measurement result;
- a control unit configured to control operation of the measurement unit;
- an data exchange interface configured to receive data from the writable memory and to forward data to the sensor module for storage in the writable memory; and
- a communications interface for communicating with a remote host system;
wherein the method comprises performing the following steps by the control unit:
a) receiving, from the remote host system via said communications interface, one or more operational parameters associated with the sensor module;
b) causing writing, via said data exchange interface, the received one or more operational parameters to the writable memory; and
c) controlling operation of the measurement unit in accordance with the received one or more operational parameters.

Accordingly, as the operational parameters are received from the remote host system after the sensor module has been received by the receptacle of the apparatus, the risk of using outdated operational parameters for controlling operation of the measurement unit is considerably reduced. This may be particularly important when operational parameters to be used with a certain sensor module have changed between the time of manufacturing of the sensor module and the time of taking the sensor module in use. For example, experiences from use of a particular type of sensor module may have shown that certain parameter values are more suitable than originally specified parameter values of the operational parameters. Embodiments of the method described herein thus provide a mechanism for facilitating use of updated operational parameters. In some situations it may even have turned out that a certain type or batch of sensor modules should be recalled and not used for making measurements, or only used with certain restrictions. The received operational parameters may thus even indicate such a recall of the sensor module or usage restrictions. Hence, a mechanism is provided that increases the reliability of the measurement results of an apparatus for analyzing biological samples which uses a replaceable sensor module.

Embodiments of the method disclosed herein further facilitate the implementation of customer-specific and/or apparatus-specific operational parameters associated with different sensor modules without having to manufacture different types of sensor modules and/or having to keep multiple types sensor modules in stock. The customer-specific and/or apparatus-specific operational parameters may simply be written to the writable memory of the sensor module when the sensor module is taken in use, i.e. the customization of the sensor module does not need to occur prior to taking the sensor module in use. Examples of such operational parameters include a maximum remaining number of samples that can be analyzed with the sensor module or a remaining maximum duration of use. The sensor modules may be manufactured for a sufficiently large maximum number of tests and then configured by the analyzer to the specific number of samples that corresponds to the contractual agreement between the supplier and the customer. Accordingly, the customer of the analyzers and the sensor modules experiences that the sensor modules last precisely as long as they need and thereby reduce the waste (throwing out unused test, or changing too many sensor modules). Moreover, the need for inventory is reduced as the number of required variants of sensor modules to be kept in stock may be significantly reduce.

As the received one or more operational parameters are written to the writable memory of the replaceable sensor module, the operational parameters are robustly retrievable during a variety of operational situations, e.g. including situations where the analyzer is not on-line, i.e. cannot communicate with a remote host system, and/or in situations where the replaceable sensor module is removed from an analyzer and inserted into another analyzer, e.g. an analyzer that is temporarily or permanently offline, i.e. cannot communicate with the remote system.

Accordingly, embodiments of the method disclosed herein facilitate a reliable, safe, robust and low-cost method of operating an apparatus for analyzing biological samples that uses a replaceable sensor module.

The apparatus for analyzing biological samples will also be referred to as an analyzer. The analyzer may be an analyzer for measuring physical parameters of analytes in a biological sample, such as in a body fluid or other liquid biological sample.

Examples of biological samples may include fluid samples, such as liquid samples and/or gas samples. Liquid samples may be selected from the group of blood, diluted or undiluted whole blood, serum, plasma, saliva, urine, cerebrospinal liquid, pleura, synovial liquid, ascites liquid, peritoneal liquid, amniotic liquid, milk, dialysis liquid samples, or the like, as well as any quality control materials and calibration solutions used in analyzer equipment for measuring any of these fluids. Gaseous samples may include respirator gas, expiratory air, or the like, as well as any quality control and calibration materials used in analyzer equipment for measuring any of these fluids. The sample may be treated prior to testing in order to make it more amenable to being tested. Pretreatment methods may include dilution, filtration, concentration, extraction, removal or inactivation of components which might interfere with the results, and addition of reagents. Examples of other biological samples include fermentation broths or microbial cultures, waste water, food products, and the like.

Further, according to some embodiments, the biological sample is a liquid, such as a body liquid, i.e. a physiological liquid. Correspondingly, a sample analyzer for use in performing the method may be adapted to analyzing parameters of liquid samples, such as body liquids, i.e. physiological liquids. A medical sample analyzer may thus include a liquid handling system comprising valves, conduits, and/or pumping/transfer means, for controlling liquid flow, such as for filling and emptying of the measurement chamber with the liquid sample - preferably in an automated manner.

Further, according to some embodiments, the fluid sample is a liquid sample selected from the group of blood, diluted or undiluted whole blood, serum, plasma, saliva, urine, cerebrospinal liquid, pleura, synovial liquid, ascites liquid, peritoneal liquid, amniotic liquid, milk, dialysis liquid samples, or the like, as well as any quality control materials and calibration solutions used in analyzer equipment for measuring any of these fluids.

Further, according to some embodiments, the fluid sample is a gas, e.g. a medical gas, such as a physiological gas. Correspondingly, a sample analyzer for use in the method is advantageously adapted to analyzing parameters of medical gas samples. Examples of particularly useful medical gas samples are selected from the group of respirator gas, expiratory air, or the like, as well as any quality control and calibration materials used in analyzer equipment for measuring any of these fluids. A medical sample analyzer may thus include a gas handling system comprising valves, conduits, and/or pumping/transfer means, for controlling gas flow, such as for filling and emptying of the measurement chamber with the gas sample - preferably in an automated manner. A medical sample analyzer may even comprise a fluid handling system suited for both liquid and gas.

In some embodiments, the analyzer is a blood analyzer configured to provide measurements of various parameters for analyzing the blood of a mammal subject, e.g. for establishing and/or monitoring a biological condition of the subject. Typically, the mammal subject is a human patient. The analyzer may be configured to measure one or multiple physical parameters of a blood sample, e.g. the partial pressure of one or more blood gasses in a whole blood sample of the mammal subject, concentrations of one or more electrolytes and/or metabolites in the blood sample, and/or the hematocrit value of the blood sample. For example, the blood analyzer may be configured to measure one or more of the following parameters: *p*CO₂, *p*O₂, *p*H, Na⁺, K⁺, Ca²⁺, Cl⁻, Mg²⁺ glucose, lactate, creatinine, urea and hemoglobin and hemoglobin-derivate values, as these parameters are important clinical indications in assessing the condition of a medical patient. It will be appreciated that some embodiments of an analyzer may only be configured to measure one or some of the above parameters while other embodiments may be configured to measure all of the above parameters. It will further be appreciated that some embodiments of an analyzer may be configured to measure alternative and/or additional parameters.

Sensor technologies and sample handling systems suitable for analyzing various types of biological samples are known as such in the art and will not be described in detail.

Embodiments of the replaceable sensor module may be a sensor cassette or other replaceable module comprising one or more sensors. In some embodiments, the sensor module includes additional components, e.g. electronic devices, containers with consumables, etc. Some embodiments of analyzers include two or more separately replaceable modules, e.g. a sensor cassette and one or more separately replaceable containers comprising consumables, e.g. substances for use for cleaning the measurement unit, for calibrating and/or quality control of the measurement unit and/or the like. In other embodiments, the sensor module itself may comprise one or more containers for accommodating consumables. Accordingly, the term sensor module as used herein is intended to comprise sensor cassettes that only include the sensors and no additional consumables as well as combined modules that include sensors as well as consumables and/or other additional components. It will further be appreciated that yet further types of replaceable sensor modules may be provided which are also intended to be encompassed by the term sensor module.

The measurement unit of the apparatus may comprise any suitable mechanism and components for bringing the biological sample into operational interaction with at least a first sensor of the one or more sensors of a sensor module received by said receptacle, for obtaining a measurement result responsive to the interaction between at least the first sensor and the biological sample and for outputting the obtained measurement result.

For example, the measurement unit may include a sample input port for receiving a biological sample, e.g. from a sample holder, a sample container etc. The measurement unit may include a suitable sample conveyance mechanism, e.g. including conduits, movable parts, etc. for bringing the biological sample into operational interaction with at least a first sensor of the one or more sensors of a sensor module and/or for subsequently dispensing the analyzed sample or for accommodating the analyzed sample in a waste container. To this end, the analyzer may include a suitable fluid handling system, e.g. as described above.

The measurement unit may further comprise a suitable signal processing unit for receiving sensor signals from the one or more sensors and for processing the received signals, e.g. for filtering, amplifying, converting the signals and/or the like. The measurement unit may further comprise a suitable signal analysis unit for analyzing the, optionally processed, sensor signals so as to obtain a measurement result. The signal processing and signal analysis units may be separate modules or they may be integrated into a single module e.g. into a single signal and data processing unit. The signal-processing unit and/or the control unit may be located in the measurement unit or in the apparatus.

The measurement unit may include a user-interface, e.g. including a display, a printer and/or the like, configured to convey operational information to the user, e.g. values of the received operational parameters, usage restrictions represented by the operational parameters, measurement results, error messages, etc. Alternatively or additionally, the apparatus may include another type of output interface, e.g. for communicating such information to a data processing system, e.g. a healthcare facility information system, e.g. via a computer network.

The control unit may completely or partly be integrated with the signal processing and/or signal analysis unit or it may be implemented as a separate unit. The control unit may be implemented by a programmable processing unit, e.g. including one or more microprocessors, or any other suitable circuitry or device for controlling operation of the apparatus.

The sensor module may include one or multiple sensors, e.g. sensors using different sensing technologies for determining different parameters. The one or more sensors may use any suitable sensing technology useful for analyzing biological samples. For example, the sensor may be an optical sensor or it may rely on another form of physical and/or chemical interaction between the sample and the sensor. Examples sensors may be configured to determine one or more of a variety of parameters, e.g. one or more of the following parameters: *p*O₂, *p*CO₂, *p*H; concentrations of electrolytes such as Li⁺, Na⁺. K⁺, Ca²⁺, Mg²⁺, Cl⁻, HCO³⁻ or NH₃(NH₄⁺); concentrations of metabolic factors, such as glucose, creatinine, urea (BUN), uric acid, lactic acid, pyruvic acid, ascorbic acid, phosphate or protein; concentrations of enzymes such as lactic acid dehydrogenase, lipase, amylase, choline, esterase, alkaline phosphatase, acid phosphatase, alanine amino transferase, aspartate, amino transferase, or creatinine kinase.

The writable memory may use any suitable type of memory technology for storing data, e.g. an EPROM, EEPROM, a writable RFID tag, etc. It will be appreciated that the choice of memory technology may depend on the amount of data to be written, the requirements for the number of times the memory is to be re-written, and/or on other factors, such as unit price, energy consumption, etc. In some embodiments the writable memory may be a one-time writable memory while, in other embodiments, the memory may be rewritable, i.e. rewritable multiple times. The sensor module may further include memory control logic configured to perform the writing and/or reading operations. Hence, causing, by the control unit of the analyzer, writing the operational parameters may comprise requesting or controlling the memory control logic of the sensor module to perform the writing. In other embodiments, causing, by the control unit of the analyzer, writing the operational parameters may comprise performing the write operation by the analyzer itself.

The data-exchange interface may use any suitable data exchange technology e.g. data exchange via a galvanic contact or a contactless data exchange. Examples of contactless data exchange include data exchange via a capacitive coupling, an inductive coupling, a near-field communications interface, a short-range wireless communications interface, e.g. a radio-frequency communications interface, a near-infrared communications interface or any other suitable data exchange technology.

The receptacle may be any suitable device for operationally receiving the sensor module, e.g. via any suitable connector that may establish a mechanical and/or electrical and/or otherwise operational connection between the sensor module and the measurement unit. The receptacle may define a compartment that completely or partially encloses the sensor module when received by the receptacle. The compartment may include a retaining mechanism and/or a connector for mechanically and/or electrically connecting the sensor module to the apparatus. The compartment may further include a lid, cover or another mechanism for closing the compartment. Alternatively, the receptacle may simply provide a connecting mechanism to which the sensor module is connectable without being enclosed by any enclosure of the receptacle, or the receptacle may provide a partial enclosure, e.g. a slot or female connector that only receives a connecting portion of the senor module while another portion of the sensor module extends outside of the receptacle when received by the receptacle. Hence, the receptacle may be, or include, a compartment, a recess, a connector such as a female or a male connector, a docking mechanism, a slot, a drawer, and/or the like.

The communications interface for communicating with the remote host system may be a wired or wireless interface e.g. a network adaptor, a serial port, a USB interface, a parallel port, a wifi interface, a Bluetooth interface, a local area network connector, and/or another suitable interface. The apparatus may be connected to the remote host system via a suitable communications network, e.g. a local area network, an internet, a computer network, a telecommunications network and/or the like or a combination thereof.

The remote host system may be a suitably programmed and configured data processing system including one or more computers, virtual machines and/or the like. The remote host system may be a single central system or a distributed system; it may be suitably programmed to implement an analyzer management system, a customer relationship management system, a product management system, a product maintenance system, and/or the like. In some embodiments, the remote host system comprises - or is communicatively connectable to - one or more databases, e.g. a sensor module database maintaining information associated with a plurality of sensor modules, and/or an analyzer database maintaining information associated with a plurality of analyzers and/or a customer database maintaining information associated with a plurality of customers, e.g. a customer relationship management system. It will be appreciated that the remote host system may include one or more databases that allow associating customer-related information and/or analyzer-related information and/or sensor-module-related information with each other. In some embodiments the remote host system is operated by a device manufacturer or vendor, e.g. a manufacturer of analyzers and/or sensor modules. However, in other embodiments, the remote host system is operated by another entity, e.g. a health care facility, a distributor, a vendor, and/or the like.

In some embodiments, causing writing the received one or more operational parameters to the writable memory comprises causing writing a time stamp to the writable memory, the time stamp being associated with the one or more operational parameters. Accordingly, when subsequently retrieving the operational parameters from the memory, the analyzer may determine whether the operational parameters are likely up-to-date or whether the analyzer should attempt to receive updated operational parameters from the host system. Accordingly, the method may comprise requesting updated operational parameters from the remote host system based on the stored time stamp. The analyzer may even be configured to only control operation of the measurement unit in accordance with the one or more operational parameters stored on the writable memory, if the time stamp is not older than a maximum validity period.

In some embodiments, receiving the one or more operational parameters comprises:
- receiving, via the data exchange interface, a module identifier from the writable memory of the sensor module received by the receptacle;
- communicating, via the communications interface, the read module identifier to the remote host system;
- receiving, from the remote host system via the communications interface, one or more operational parameters associated with the communicated module identifier.

In particular, the analyzer may be informed by the host system about any changes in operational parameters to be used with a particular sensor module. Moreover, the host system may keep a record of when a particular sensor module has been taken in use, thus facilitating inventory management, billing etc. To this end, communicating the read module identifier may further comprise communicating a time stamp indicative of a time of reading the module identifier from the writable memory. Accordingly, the time of initial use of the sensor module may more accurately be communicated, e.g. in situations when the analyzer was off-line or otherwise unable to immediately communicate the module identifier to the remote host system. In such situations the analyzer may cache the information and communicate it to the host system the next time communication is established. The module identifier may be any suitable identifier identifying the sensor module; it may be a unique identifier uniquely identifying a particular sensor module or it may be an identifier identifying a group of sensor modules, e.g. a batch of sensor modules manufactured at a certain time, a type of sensor module, etc. In some embodiments, the module identifier is a serial number.

In some embodiments, receiving the one or more operational parameters comprises:
- communicating, via the communications interface, an analyzer identifier to the remote host system, the analyzer identifier identifying the apparatus for analyzing biological samples;
- receiving, from the remote host system via the communications interface, one or more operational parameters associated with the communicated analyzer identifier.

Accordingly, the analyzer may be informed by the host system about operational parameters to be used by the particular analyzer when operated with a sensor module. For example, the host system may, based on the analyzer identifier, retrieve analyzer-specific information. Alternatively or additionally, the host system may retrieve information about which customer is operating the identified analyzer and retrieve customer-specific operational parameters, such as customer-specific preferences, customer-specific usage restrictions, etc. In particular, this method allows the supplier to implement a customer-specific billing scheme where the customer is billed for a predetermined usage pattern - e.g. usage time and/or number of samples to be analyzed - of a sensor module. The host system may then communicate one or more operational parameters indicative of such usage pattern or restriction to the analyzer. The analyzer identifier may be any suitable identifier identifying the analyzer; it may be a unique identifier uniquely identifying a particular analyzer or it may be an identifier identifying a group of analyzers, e.g. all analyzers of a particular (part of an) organization, a particular type of analyzer, etc. In some embodiments, the analyzer identifier is a serial number. Alternatively or additionally, the remote host system may maintain a usage history of the analyzer, e.g. based on log data received from the analyzer. The remote host system may thus determine one or more operational parameters based on the stored usage history. For example, the remote host system may set the maximum remaining number of samples to be analyzed by the sensor module based on the typical historic use pattern. This information may further be used for billing purposes.

As the operational parameters are written to the memory of the sensor module, the usage pattern and/or restriction for a sensor module may be enforced even if the sensor module is subsequently used with another analyzer. Accordingly said one or more operational parameters associated with the communicated analyzer identifier may include a usage restriction parameter indicative of a usage restriction associated with the sensor module when used with the apparatus for analyzing biological samples identified by the analyzer identifier. Examples of usage restrictions may include a restriction of use of a sensor module only with a particular set of one or more analyzers, a restriction of a maximum remaining number of samples to be analyzed with a sensor module, a restriction of a maximum remaining period of time a sensor module may be used, a restriction as to which types of analyses a sensor module can perform, and/or the like. In some embodiments, the one or more operational parameters associated with the sensor module include a module expiration parameter indicative of an operational lifetime and/or of a maximum remaining number of samples associated with the sensor module. In particular, in some embodiments, controlling operation of the measurement unit in accordance with the received one or more operational parameters includes preventing further measurements by the measurement unit with a current sensor module received by the receptacle when the operational lifetime or the maximum remaining number of samples associated with the current sensor module received by the receptacle has been exceeded. Accordingly, when a sensor module is inserted into the analyzer, the maximum remaining number of samples may be determined by correlating the analyzer with online data about the historic usage pattern and/or online data about the contractual agreements between the customer and the vendor.

The receipt of the operational parameters from the remote host system and the writing of these parameters to the writable memory of the sensor module may not need to be performed each time a measurement is to be performed or even each time the apparatus is started up, e.g. powered up or started from a stand-bye mode. In particular the receipt and writing of the operation parameters may, in some embodiments, only be necessary the first time a sensor module is taken in use, e.g. the first time the sensor module is operationally connected to an analyzer.

Accordingly, in some embodiments, the method may comprise performing the following steps by the control unit:
- upon start-up of the apparatus, receiving information, via the data exchange interface, from the writable memory of a sensor module received by the receptacle;
- determining, based on the received information, whether or not the sensor module has previously been used;
- responsive to determining that the sensor module has not previously been used, performing steps a) through c); otherwise controlling operation of the measurement unit in accordance with one or more previously stored operational parameters read from the writable memory via the data exchange interface.

The information received from the writable memory may e.g. include a time stamp indicative of a time of writing operational parameters and/or a flag indicating whether parameters have previously been written to the memory and/or one or more of the operational parameters themselves. In particular, upon manufacture of the sensor module, such time stamp and/or flag and/or one or more parameter values of the one or more operational parameters may be set to a default value, e.g. zero or another value that is not a valid time stamp or parameter value etc. as the case may be. When writing the operational parameters to the writable memory, the analyzer may set the time stamp to the actual time of writing and/or set the flag and/or set the parameter values to actual values. Hence, if the received information reflects a default value, the analyzer may determine that the sensor module has not been previously been used.

Optionally, the analyzer may subsequently, e.g. periodically and/or in response to a user input or another type of trigger event, request updated operational parameters from the remote host system.

If, upon start-up or when triggered to request updated operational parameters, no communication can be established to the remote host system, it may be desirable that the analyzer is operational, even if the apparatus would normally be configured to receive (updated) operational parameters. Such situation may e.g. occur when the analyzer is offline e.g. due to computer network problems or for other reasons. If the sensor module has stored thereon previously written operational parameters, the analyzer may of course read these parameters and control operation of the measurement unit based on these previously written parameters.

Accordingly, in some embodiments, the method comprises performing the following steps by the control unit:
- upon start-up of the apparatus, determining whether or not communication with the remote host system can be established via the communications interface;
- responsive to determining that communication with the remote host system can be established, performing steps a) through c); otherwise controlling operation of the measurement unit in accordance with one or more previously stored operational parameters read from the writable memory via the data exchange interface.

If no communication can be established to the remote host system when a new sensor module is used for the first time, the analyzer may, in some embodiments, read default operational parameters from the memory of the new sensor module that had been stored in the memory during manufacturing or during subsequent configuration of the sensor module. Alternatively, the analyzer may use a set of default parameters stored in a memory of the analyzer. In some embodiments, the analyzer may only allow operation based on such default parameters form a certain period of time and/or for a certain number of samples.

In some embodiments, the method comprises:
- maintaining, in a data storage of the apparatus, a local cache of one or more cached operational parameters;
- responsive to determining that the sensor module has not previously been used and that communication with the remote host system cannot be established, causing writing, via said data exchange interface, the cached operational parameters to the writable memory; and controlling operation of the measurement unit in accordance with the cached one or more operational parameters.

In particular, in some embodiments, the method comprises, responsive to determining that communication with the remote host system can again be established:
- receiving, from the remote host system via said communications interface, one or more updated operational parameters associated with the sensor module;
- causing writing, via said data exchange interface, the received one or more updated operational parameters to the writable memory; and
- controlling operation of the measurement unit in accordance with the received one or more updated operational parameters.

In some embodiments, steps a) through c) are performed responsive to detecting that a new sensor module has been inserted into the receptacle. Alternatively or additionally, in some embodiments, the method comprises performing the following steps by the control unit:
- controlling the measurement unit to perform one or more quality control measurements;
- responsive to a result of the one or more quality control measurements, obtaining one or more modified operational parameters associated with the sensor module received by the receptacle;
- causing writing, via said data exchange interface, the one or more modified operational parameters to the writable memory; and
- controlling operation of the measurement unit in accordance with the one or more modified operational parameters.

The quality control measurements may e.g. be measurements performed on one or more predetermined quality control or calibration samples such that the measurement results may be compared with expected measurement results. Dependent on an outcome of the quality control measurement, the analyzer may modify the operational parameters to be used with the particular sensor module and write the modified operational parameters to the writable memory, thus improving subsequent measurement performance of the analyzer. Alternatively, the analyzer may communicate the result of the one or more quality control measurements to the remote host system which may then determine one or more modified operational parameters associated with the sensor module. The analyzer may thus receive the one or more modified operational parameters from the remote system, cause writing, via said data exchange interface, the received one or more modified operational parameters to the writable memory; and control operation of the measurement unit in accordance with the one or more modified operational parameters. Accordingly, production and/or quality data may be used in the determination of the operational parameters (e.g. of the maximum number of samples to be analyzed), thereby reducing the experienced in field error-rates.

In some embodiments, the method comprises causing, by the control unit, responsive to operation of the measurement unit, writing usage data, such as log data, to the writable memory via the data exchange interface, the usage data being indicative of one or more usage parameters. Alternatively or additionally, the method may comprise, responsive to operation of the measurement unit, communicating, by the control unit via said communications interface, usage data to the remote host system, the usage data being indicative of one or more usage parameters. Examples of usage data may include log data, e.g. the number of samples analyzed by the sensor module or time stamps of measurements. The usage data may even include measurement results, or a combination of log data and measurement results. Accordingly, based on the thus written usage data, the analyzer and/or the remote host system may e.g. subsequently determine whether usage restrictions have been exceeded, may invoke billing, may control inventory management and/or react in a different manner. In some embodiments, the remote host system may use the usage data to determine operational parameters for subsequent sensor modules. In some embodiments, the analyzer may log the usage data on the writable memory of the sensor module and periodically, e.g. in predetermined time intervals or when the analyzer is online, communicate the logged usage data to the remote host system.

In some embodiments, the one or more operational parameters and/or usage data are written to the writable memory in cryptographically encrypted form. Alternatively, the one or more operational parameters and/or usage data may be written to the writable memory in cryptographically authenticated form. Accordingly, unauthorized access to the stored data may be prevented and/or it may be ensured that the data is not tampered with or otherwise altered in an unauthorized fashion.

In some embodiments, the one or more operational parameters associated with the sensor module include one or more parameters chosen from:
- a lock/unlock flag indicative of whether the control unit is to allow measurements to be performed with the sensor module;
- one or more feature lock/unlock flags indicative of whether the control unit is to lock or unlock one or more selected features of the apparatus with the sensor module received by the receptacle;
- one or more usage restriction parameters restricting usage of the sensor module to one or more locations and/or operators and/or health care facilities;
- one or more start-up parameters indicative of a required start-up configuration of the apparatus with the sensor module received by the receptacle;
- one or more measurement parameters indicative of a required measurement configuration of the apparatus with the sensor module received by the receptacle.

The present disclosure relates to different aspects including the method described above, corresponding apparatus, systems, methods, and/or products, each yielding one or more of the benefits and advantages described in connection with one or more of the other aspects, and each having one or more embodiments corresponding to the embodiments described in connection with one or more of the other aspects and/or disclosed in the appended claims.

In particular, according to one aspect, an apparatus for analyzing biological samples comprises:
- a receptacle for receiving a replaceable sensor module, the sensor module comprising one or more sensors and a writable memory;
- a measurement unit configured to bring a biological sample into operational interaction with at least a first sensor of the one or more sensors of a sensor module received by said receptacle, to obtain a measurement result responsive to the interaction between at least the first sensor and the biological sample and to output the obtained measurement result;
- a control unit configured to control operation of the measurement unit;
- an data exchange interface configured to receive data from the writable memory and to forward data to the sensor module for storage in the writable memory; and
- a communications interface for communicating with a remote host system;
wherein the control unit is configured to perform the steps of the method disclosed above and in the following.

Generally, here and in the following the term control unit is intended to comprise any circuit and/or device and/or system suitably adapted to perform the functions described herein. In particular, the above term comprises general- or special-purpose programmable microprocessors, such as a central processing unit (CPU) of a computer or other data processing device or system, Digital Signal Processors (DSP), Application Specific Integrated Circuits (ASIC), Programmable Logic Arrays (PLA), Field Programmable Gate Arrays (FPGA), special purpose electronic circuits, etc., or a combination thereof. The control unit may be implemented as a plurality of processing units.

According to another aspect, a computer program product comprises executable program code configured to cause a control unit of an apparatus for analyzing biological samples to perform the steps of the method disclosed above and in the following, when the program code is executed by the control unit. The computer program product may be provided as a computer-readable medium, such as a CD-ROM, DVD, optical disc, memory card, flash memory, magnetic storage device, floppy disk, hard disk, etc. In other embodiments, a computer program product may be provided as a downloadable software package, e.g. on a web server for download over the internet or other computer or communication network.

According to yet another aspect, a system for analyzing biological samples comprises:
- an apparatus as defined herein;
- a replaceable sensor module comprising one or more sensors and a writable memory; and
- a remote host system described herein, configured to send one or more operational parameters associated with the sensor module to the apparatus.

According to yet another aspect, the host system comprises:
- a communications interface for communicating with one or more apparatus for analyzing biological samples;
- a data processing unit; and
- a database comprising data indicative of one or more customer-specific operational parameters and/or of one or more module-specific operational parameters;
wherein the data processing unit is configured to:
- retrieve from the database, responsive to receipt of a module identifier and/or an analyzer identifier from an apparatus for analyzing biological samples via the communications interface, the one or more customer-specific operational parameters and/or the one or more module-specific operational parameters, and to
- forward the retrieved one or more customer-specific operational parameters and/or the one or more module-specific operational parameters to the apparatus for analyzing biological samples via the communications interface.

The host system may be embodied as a data processing system. It may comprise or be connectable to a computer-readable medium from which a computer program can be loaded into the processing unit, such as a CPU, for execution. The computer-readable medium may thus have stored thereon program code means adapted to cause, when executed on the data processing system, the data processing system to perform steps of the method described herein. The data processing system may comprise a suitably programmed computer, a system of multiple computers, a virtual machine, or another programmable computing device. The host system may be connected to a suitable communications network such as the internet.

In some embodiments, the data processing unit is further configured to:
- receive usage data from the apparatus, the usage data being indicative of one or more usage parameters of the apparatus.
- store the received usage data in the database;
- determine the one or more customer-specific operational parameters and/or the one or more module-specific operational parameters based on the stored usage data.

According to yet another aspect, a computer program product comprises program code configured to cause a data processing unit of the host system to perform the following steps when the program code is executed by the data processing unit:
- retrieving, responsive to receiving, via a communications interface of the host system, a module identifier and/or an analyzer identifier from an apparatus for analyzing biological samples, one or more customer-specific operational parameters and/or one or more module-specific operational parameters from a database associated with the host system;
- forwarding the retrieved one or more customer-specific operational parameters and/or the one or more module-specific operational parameters to the apparatus for analyzing biological samples via the communications interface.

As above, the computer program product according to this aspect may be provided as a computer-readable medium, such as a CD-ROM, DVD, optical disc, memory card, flash memory, magnetic storage device, floppy disk, hard disk, etc. In other embodiments, a computer program product may be provided as a downloadable software package, e.g. on a web server for download over the internet or other computer or communication network.

### BRIEF DESCRIPTION OF THE DRAWINGS

Embodiments of the various aspects disclosed herein will be described in more detail in connection with the appended drawings, which show in
- Fig. 1: a block diagram of a system comprising remote host system and a blood analyzer with a replaceable sensor module;
- Fig. 2A an: example of a memory layout of a writable memory of a replaceable sensor module;
- Fig. 2B: another example of a memory layout of a writable memory of a replaceable sensor module;
- Fig. 3: a flow diagram of an embodiment of a process for operating an analyzer for analyzing biological samples;
- Fig. 4: a data flow diagram of an embodiment of a process for updating operational parameters associated with a replaceable sensor module;
- Fig. 5: a flow diagram of an embodiment of a process for performing a measurement by an analyzer for analyzing biological samples;
- Fig. 6: a flow diagram of an embodiment of a process for performing a quality-control measurement by an analyzer for analyzing biological samples.

### DETAILED DESCRIPTION

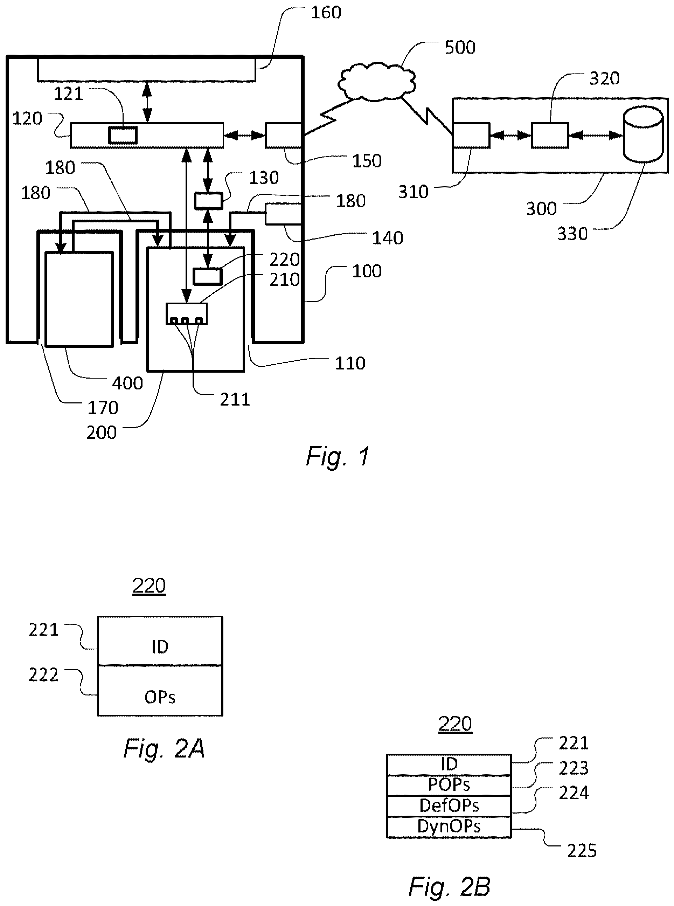

Fig. 1 shows a block diagram of a system comprising sample analyzer 100, a replaceable sensor module 200 and a remote host system 300. In this example, the sample analyzer 100 is a blood analyzer. It will be appreciated though that, in other embodiments, the sample analyzer may be an analyzer for analyzing other types of biological samples. The sample analyzer 100 comprises a receptacle 110 for receiving the sensor module 200, e.g. a sensor cassette. In the present example, the analyzer comprises a further receptacle 170 for receiving a container module 400 including one or more reservoirs accommodating consumables, such as process liquids for rinsing, calibration and/or quality control purposes, and a waste reservoir. It will be appreciated that alternative embodiments of an analyzer may be operable without any need for a separate container module. For example, the sensor module may also include one or more consumables. In yet further alternative embodiments, the analyzer may include a single receptacle for receiving a sensor module as well as a container module.

The analyzer comprises a sample inlet port 140 for receiving a sample to be analysed.

The sensor module 200 comprises one or more sensors 211 for analyzing a sample received by the analyzer via the sample inlet port 140. For example, the sensors may be deposited in a measurement chamber 210.

The analyzer 100, the sensor module 200 and the container module 400 comprise a sample handling infrastructure 180 for conveying a received sample into operational interaction with the sensors 211, for convening any consumables from the container module 400 and for transporting any waste to the container module. The specific details of the sample handling infrastructure may depend on the nature of the samples to be analyzed. For example, in case of blood samples or similar liquid samples, the sample handling infrastructure may comprise suitable conduits connecting the sample inlet port 140 with the sensor module 200 and for connecting the sensor module 200 with the container module 400, as well as one or more pumps and/or other liquid handling components, e.g. valves. The conduits may comprise suitable conduit coupling mechanisms allowing the establishment of fluid communication between the analyzer and the sensor module and between the analyzer and the container module.

During operation, a sample is transferred through the inlet port 140 to the measurement chamber 210 comprising the sensors 211. The sensors 211 are arranged to provide essentially simultaneous measurements on analyte parameters in a sample, e.g. in a whole blood sample. Preferably, the required sample amount for obtaining precise and reliable data is as small as possible. A detailed example of a sensor assembly design that is particularly suitable for simultaneously measuring a plurality of different parameters in bodily fluids, particularly in whole blood, and its use in a blood analyzer is e.g. found in EP 2 147 307 B1.

The analyzer 100 further comprises a control unit 120 including a signal and data processing unit 121, e.g. including a suitably programmed central processing unit. The control unit is configured to control operation of the analyzer, including controlling operation of the sample handling infrastructure. The control unit further receives sensor signals from the sensors, processes the received sensor signals to compute measurement results and to output the computed measurement results. To this end, the control unit is operationally coupled to a user interface 160. The user interface 160 may include a display and user input elements, e.g. physical input elements such as buttons and/or virtual input elements implemented via a touch screen or the like.

During operation, based on pre-programmed instructions loaded in the signal and data processing unit 212 and, optionally, based on user input, the analyzer performs measurements on a received sample using the sensors 211. The sensors 211 generate sensor signals that are representative of a physical parameter for respective analytes and provide the sensor signals to the signal and data processing unit 121. The signal and data processing unit 121 is adapted to receive and process sensor signals from the sensors 211, and to present the processed signals as output to a user, e.g. via user interface 160, and/or forward the output to a subsequent/further data analysis. After measurement, the sample is discharged in a waste reservoir of the container unit 400, and the measurement chamber 210 is prepared for the next measurement.

The embodiment of the analyzer shown in Fig.1 is particularly adapted for the measurement of blood parameters. Performing the measurements, calibration tasks, and quality control procedures thus typically involves the loading, unloading, rinsing, cleaning and re-loading of different liquids, which may be done by the fluid handling infrastructure 180 using consumables provided in the container unit 400. The fluid handling may be controlled in an automated way by the control unit 120 according to pre-programmed instructions and/or user input. The container module may include a number of reservoirs pre-filled with process liquids for rinsing/wash-out, calibration and quality control tasks. The process liquids have a known composition. The process liquid for a given process step may be selected by a fluid selector valve and transferred via a feed line into the measurement chamber. The discharged fluids are finally transported through a fluid line to the waste reservoir of the container module 400.

Upon start-up and/or in an ongoing manner during uptime, the analyzer 100 may be configured to perform self-control routines. If any abnormality is detected, the analyzer 100 indicates the deviation to a user via the user interface 160, and may further indicate ways of overcoming an error state. On the other hand, when the analyzer indicates normal operation, measurements can be performed immediately. Advantageously, according to some embodiments, the self-control routines may be performed during idle times, i.e. when the analyzer is in an idle state, where it is not used for performing actual measurements on a user's sample. The self-control routines may include continued repetitive measurements performed on a calibration-grade and/or quality-control process liquid with a precisely known composition. The signals obtained for each of the different analyte sensors 211on the known composition may then be used to continuously update the reference for the respective analyte measurements and/or to modify one or more operating parameters associated with the currently used sensor module.

The sensor module 200 has associated with it a number of operational parameters. In particular, the sensor module may have associated with it a maximum remaining number of samples that can be analyzed by the sensor module, e.g. a maximum number of samples that can be analyzed without undue degradation of the sensors and/or based on a contractual agreement with the sensor module vendor. In some embodiments the maximum remaining number of samples may be determined by a contractual relationship between the analyzer supplier and the customer, i.e. the entity using the analyzer, e.g. a healthcare facility. For example, a unit prize of the sensor module may depend on the maximum number of samples that can be analyzed by the sensor module. Alternatively or additionally the sensor module may have an operational lifetime associated with it, e.g. a maximum remaining period of time the sensor module may be kept in operation once it has been taken in use. Again, the operational lifetime may depend on the properties of the sensors and/or on contractual relationship between the supplier and the customer. Alternatively or additionally, the maximum remaining number of samples or the operational lifetime may be determined in dependence of stored information about the historic usage pattern of the analyzer. To this end, information about the historic usage pattern of the analyzer may be stored by the remote host system.

Another example of an operational parameter may include the shelf life of the sensor unit, e.g. a total lifetime of the sensor unit from the time of its manufacturing, irrespective of whether it has been used or not. Yet further operational parameters may include operational parameters to be used by the analyzer with a particular sensor module, e.g. the selection of process fluids, the frequency of rinsing and/or calibration etc.

The sensor module comprises a writable memory 220, e.g. in the form of a contactlessly readable and writable memory, e.g. a writable RFID tag or the like. The memory 220 provides a data storage for storing the operational parameters associated with the sensor module 200. The memory 220 may also have a module identifier stored thereon for identifying the sensor module.

The analyzer comprises a data exchange interface 130, e.g. an RFID reader, configured to read data from the memory 220 and to write data to the memory 220 when the sensor module is inserted in the receptacle 110 of the analyzer. It will be appreciated that the memory technology and the interface technology for reading and writing from/to the memory may vary from embodiment to embodiment. In particular different wired or contactless data exchange interfaces may be used. The data exchange interface 130 is operationally coupled to the control unit 120 so as to allow the control unit to receive data stored in the memory 220 and to send data to the memory 220 for storage.

The analyzer further comprises a communications interface 150, e.g. a network adapter, for wired and/or wireless communication between the analyzer 100 and remote host system 300. The communication may be performed via a suitable computer network 500, e.g. including a local area network, and internet, a telecommunications network, and/or the like.

The control unit 120 is configured to exchange data with the remote host system via the communications interface 150 and the computer network 500. In particular, the control unit may be configured to communicate operational data to the remote host system, e.g. including usage statistics, error messages, an analyzer identifier identifying the analyzer, etc. The control unit may further be configured to communicate data read from the memory 220 of the sensor module 200 to the remote host system, e.g. a module identifier and/or some or all of the operational parameters stored on the memory 220. The control unit is further configured to receive information from the remote host system 300, e.g. operational data to be stored in the memory 220 of the sensor module currently inserted in the analyzer. The data exchange may e.g. occur every time the analyzer is started-up, every time a new sensor module is inserted and/or at other times, e.g. at regular intervals and/or triggered by certain events during operation of the analyzer. In some embodiments, the analyzer is only operable when it is on-line, i.e. when communication with the remote host system is possible. In other embodiments, off-line operation of the analyzer is also possible, i.e. operation of the analyzer while it is not able to communicate with the remote host system. Such offline operation may be possible over indefinite periods of time or at least during a limited period of time. In such embodiments, when the analyzer is on-line again after an off-line period, data exchange may be resumed. During off-line periods, the analyzer may be configured to maintain a local cache of data to be communicated to the remote host system upon resumption of the communication. The cache may be implemented by a memory of the analyzer and/or the memory 220 of the sensor module.

The remote host system 300 comprises a communications interface 310, e.g. a network adapter, for communicating data with analyzer 100 and, optionally, with other analyzers that are connectable to the remote host system. The remote host system further comprises a processing unit 320, e.g. a central processing unit of a computer, and/or the like. The remote host system further comprises a database 330, e.g. a product management database and/or a customer relationship management database. It will be appreciated that the remote host system may include more than one database. The database may also be configured to maintain usage statistics of analyzers operated at multiple sites. The remote host system may be implemented by one or more suitably programmed computers, e.g. server computers, virtual machines, etc. It will be appreciated that the processing unit 310, the communications interface 310 and the database 330 may be implemented as a single integrated computer or in a distributed fashion, e.g. such that the database 330 is implemented by a data processing system different to the data processing system communicating with the analyzer.

Fig. 2A illustrates an example of a memory layout of a writable memory of a replaceable sensor module, e.g. of memory 220 of FIG. 1. The memory 220 has stored thereon a module ID 221 which uniquely identifies the particular sensor module. In alternative embodiments, a non-unique identifier may be used, e.g. an identifier that identifies an entire production batch of sensor modules, a particular type of module or the like. The memory 220 has further stored thereon a number of operational parameters 222, such as one or more of the following:
- a time of initial use of the sensor module;
- a module expiration parameter indicative of an operational lifetime and/or of a maximum remaining number of samples associated with the sensor module. The operational lifetime may include an absolute expiry date (e.g. based on the time of production of the module) and/or a relative expiry time relative to the time of initial use of the sensor module. Operation of the sensor module may only be recommended or even allowed if the operational lifetime has not yet expired. If the operational lifetime defines an absolute lifetime and a relative lifetime, the operation of the sensor module may only be recommended or even allowed as long as both lifetimes have not yet expired.
- a lock/unlock flag indicative of whether the control unit is to allow measurements to be performed with the sensor module;
- one or more feature lock/unlock flags indicative of whether the control unit is to lock or unlock one or more selected features of the apparatus with the sensor module received by the receptacle;
- one or more usage restriction parameters restricting usage of the sensor module to one or more locations and/or operators and/or health care facilities;
- one or more start-up parameters indicative of a required start-up configuration of the apparatus with the sensor module received by the receptacle;
- one or more measurement parameters indicative of a required measurement configuration of the apparatus with the sensor module received by the receptacle.

It will be appreciated that the module ID 221 and/or one or more of the operational parameters may be stored in a read-only fashion, i.e. such that an analyzer cannot overwrite them. However, at least some of the operational parameters may be writable at least once and, optionally, re-writable several times, by the analyzer as described herein. In some embodiments, during production, default values for some or all of the operational parameters may be stored. During use of the sensor module, the analyzer may then overwrite the default values of some or all of the operational parameters.

Fig. 2B illustrates another example of a memory layout of a writable memory of a replaceable sensor module, e.g. of memory 220 of FIG. 1. The memory 220 has stored thereon a module ID 221 as described in connection with FIG. 2A. The memory has further stored thereon a number of operational parameters 223-225. The operational parameters include permanent parameters 223 that are stored at the time of production of the sensor module at that are not changed during the lifetime of the sensor module. The operational parameters further include default parameter values 224 for a number of operational parameters and current values 225 of these operational parameters. At the time of manufacturing, the default values are stored in the memory. During use of the sensor module, the analyzer may store updated versions 225 of these parameters in the memory, but without overwriting the default values. To this end, memory areas 22, 223 and 224 may be read-only.

In some embodiments, some or all of the data stored in the memory may be cryptographically encrypted, e.g. such that the analyzer reading the data needs to use an encryption key in order to decrypt the data. The encryption key may be analyzer-specific for a particular analyzer, or it may be a common key for a set of analyzers. Alternatively or additionally, the stored data may be cryptographically authenticity protected, e.g. digitally signed by the entity having written the data.

In some embodiments, each operational parameter is associated with a time stamp indicative of the time of storing the corresponding parameter in the memory.

It will be appreciated that the memory 220 may further be configured to store additional data, e.g. usage statistics and/or other usage data associated with the sensor module. For example, the memory may have stored thereon a counter indicating the number of samples analyzed by the sensor module.

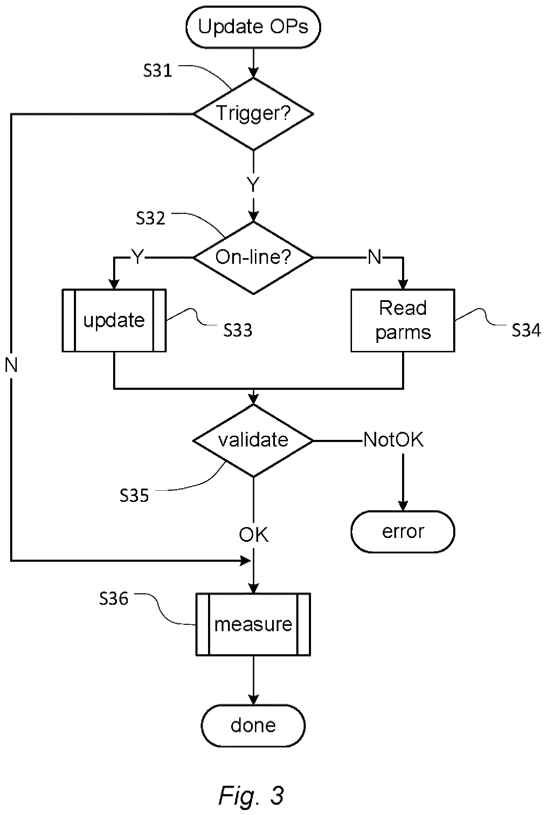

Fig. 3 illustrates a flow diagram of an embodiment of a process for operating an analyzer for analyzing biological samples, e.g. the analyzer shown in FIG. 1. In particular, the process may be performed under the control of the control unit of the analyzer.

In initial step S31, the process determines whether the operational parameters stored in the memory of the sensor module accommodated by the receptacle of the analyzer need to be updated and whether the analyzer has updated operational parameters available.

For example, in some embodiments, the operational parameters are updated each time the analyzer is started up. Alternatively or additionally, the operational parameters are updated each time a new sensor module is inserted in the receptacle of the analyzer. Alternatively or additionally, the operational parameters are updated each time a quality control measurement is performed. Alternatively or additionally, the operational parameters are updated periodically, e.g. at regular time intervals. Alternatively or additionally, the operational parameters are updated responsive to one or more other trigger events, such as responsive to a user input, responsive to establishment of a communications link with a remote host system, responsive to a request from a remote system and/or the like.

In any event, if the process determines that the operational parameters require updating or that the analyzer requires updated operational parameters for the sensor module, the process proceeds at step S32; otherwise the process proceeds at step S36.

At step S32, the process determines whether communication with a remote host system, e.g. host system 300 of FIG. 1, can be established. If this is the case, the process proceeds at step S33; otherwise the process proceeds at step S34.

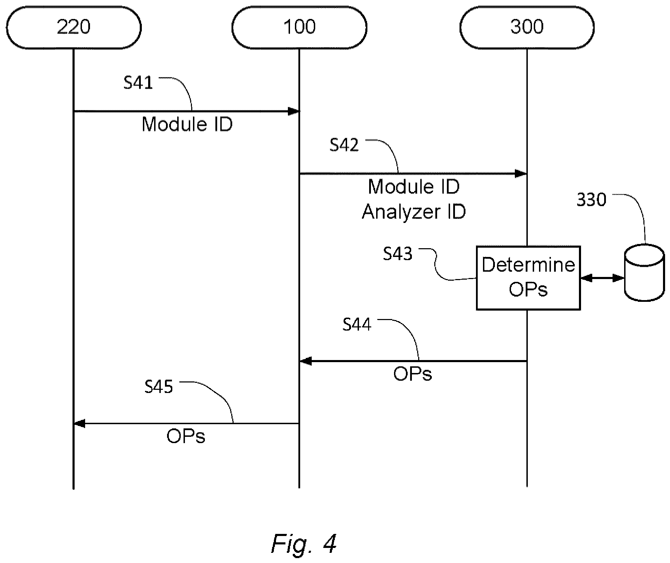

At step S33, the process updates the operational parameters stored in the memory of the sensor module. An example of the update process will be described below with reference to FIG. 4. The process then proceeds at step S35.

At step S34, the process reads the currently stored operational parameters from the memory of the sensor module. The currently stored parameters may e.g. be default parameters stored at the time of manufacturing the sensor module or they may be previously stored operational parameters, e.g. parameters stored by the same analyzer during a previous parameter update process or parameters stored by another analyzer if the sensor module has previously been inserted and used with another analyzer. The process then proceeds at step S35.

At step S35 the process validates the operational parameters obtained in step S33 or step S34. The validation may include one or more verification steps. For example:
- When the operational parameters include a maximum remaining number of samples, the process may determine the number of samples that have already been analyzed with the current sensor module. For example, this number may be obtained from a counter value stored in the memory of the sensor module.
- When the operational parameters include expiration data, e.g. an expiration date or a maximum remaining usage period from the time of first use of the sensor module, the process may determine whether the expiration date or period has been exceeded.
- When the operational parameters are authenticity protected, the process may verify whether the operational parameters have been written by an authorized source, e.g. by verifying a digital certificate, optionally based on a request to a remote host system.
- When the operational parameters include other usage restrictions, e.g. a restriction to be used only with certain types of analyzers, a restriction to be used only with one or more specific analyzers, etc., the process may compare these restrictions with the an identifier of the current analyzer and/or with other validation information.
- When the operational parameters include a recall flag, the process may determine that the sensor module may no longer be used.

It will be appreciated that the validation may include alternative or additional verification steps. In any event, the validation results in a decision as to whether or not the analyzer may proceed to perform measurements with the currently inserted sensor module and, optionally, whether the analyzer may proceed to perform measurements with the currently inserted sensor module only with certain limitations, e.g. after a prolonged warm-up time, with an extended exposure time, and/or the like.

If the validation steps results in a determination that (further) measurements with the currently inserted sensor module are not possible/advisable, the process may terminate with an appropriate error message. Otherwise the process may proceed at step S36, optionally accompanied with an appropriate indication of any applicable restrictions, if any.

At step S36, the process proceeds by performing one or more measurements of one or more biological samples.

Fig. 4 illustrates a data flow diagram of an embodiment of a process for updating operational parameters associated with a replaceable sensor module. In particular, FIG. 4 shows a possible embodiment of step S33 of the process of FIG. 3.

In initial step S41, the analyzer 100 reads a module identifier from the memory 220 of the sensor module currently inserted in the analyzer. Optionally, the analyzer may further read additional information, e.g. a time stamp or flag indicating whether or not the stored operational parameters in the memory have previously been written or updated.

In step S42, the analyzer 100 communicates the read module identifier and an analyzer identifier to the remote host system 300. The analyzer identifier may uniquely identify the particular analyzer 100 or at least identify a certain type of analyzer.

In step S43, the remote host system determines one or more operational parameters to be associated with the sensor module currently inserted into the analyzer. To this end, the host system may retrieve the corresponding operational parameter(s) from a database 330 or several databases.

For example, based on the module ID, the remote host system may query a product management database or production database that has stored thereon information about a plurality of sensor modules, e.g. information associated with individual sensor modules, information associated with certain production batches of sensor modules, with certain types of sensor modules and/or the like. For example, if during the time between manufacturing a sensor module and the time at which the module is taken in use by a customer, it has turned out that certain pre-stored operational parameters should be changed, these updated parameters may be stored in the database 330 and retrieved by the host system based on a module ID. In some situations, a recall of certain sensor modules may be required or desirable. This may e.g. be implemented in an efficient manner by implementing a recall flag as one of the operational parameters, or by setting a maximum remaining number of samples to be analyzed by a sensor module to zero, or by setting an operational lifetime of a sensor module to zero. The remote host system may also have stored information about which customer has purchased a certain sensor module and may then determine one or more customer-specific operational parameters, e.g. a customer-specific maximum remaining number of samples, a customer-specific operational lifetime etc. For example, such customer-specific parameters may be based on contractual agreements between the sensor module supplier and the customer. Such customer-specific parameters may be stored in a product management database or in a customer relationship management database.

Alternatively or additionally, based on the analyzer ID, the remote host system may query a product management database that has stored thereon information about a plurality of analyzers, e.g. analyzer-specific usage restrictions or usage restrictions specific for a certain combination of analyzer and sensor module. The remote host system may also retrieve information about which customer is operating a certain analyzer and retrieve customer-specific operational parameters such as customer-specific usage restrictions. To this end, the database may have stored information about which customer has purchased a analyzer, including any contractual agreements, billing information, etc. The remote host system may thus determine one or more customer-specific operational parameters, e.g. a customer-specific maximum remaining number of samples, a customer-specific operational lifetime etc. For example, such customer-specific parameters may be based on contractual agreements between the analyzer supplier and the customer. Such customer-specific parameters may be stored in a product management database or in a customer relationship management database.

Alternatively or additionally, the remote host system may retrieve a usage history of the analyzer, e.g. based on previously received and stored usage data from said analyzer.

In step S44, the remote host system returns the retrieved operational parameters to the analyzer.

In subsequent step S45, the analyzer writes the received operational parameters to the memory 220 of the sensor module or at least sends the data to the sensor module for storage in the memory 220.

It will be appreciated that, in alternative embodiments, the analyzer may forward only the module identifier or only the analyzer identifier to the remote system. The remote host system may then determine the corresponding operational parameters based only on the module identifier or only the analyzer identifier. Yet further, in some embodiments, the analyzer may forward additional information in addition to the module identifier and/or the analyzer identifier. The remote host system may then determine the corresponding operational parameters based also on such additional information, e.g. location information about a current location of the analyzer, etc.

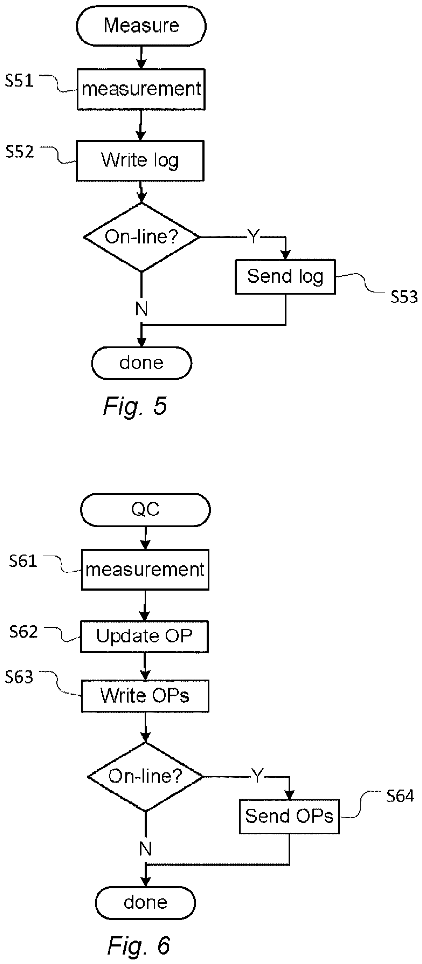

Fig. 5 illustrates a flow diagram of an embodiment of a process for performing a measurement by an analyzer for analyzing biological samples, e.g. by the analyzer of FIG. 1. In particular, FIG. 5 may represent an embodiment of step S36 of the process of FIG. 3.

In initial step S51 the process performs the measurement using the obtained operational parameters. The analyzer may display the measurement results on a display of the analyzer, generate a print-out of the results, communicate the measurement results to a health care facility information system, or the like.

In step S52 the process writes log information and/or other usage data to the memory of the sensor module currently inserted in the analyzer. Writing the log information may e.g. include, updating a counter value stored in the memory where the counter value represents the number of samples analyzed by the sensor module. The log information may include a time stamp of the measurement and/or additional log information.

If the analyzer is currently online, i.e. is able to communicate with the remote host system, the process may optionally proceed at step S53 and send log information and/or other usage data, including the information written to the memory and/or other log information or usage data, to the remote host system so as to allow the remote host system to maintain a log of the activity of the analyzer and/or the sensor module. In some embodiments, the process may synchronize the information stored in the memory with the remote log, e.g. in situations where the analyzer has been off-line, i.e. has not been able to communicate with the remote host system. For example, if the analyzer, during the off-line period, has stored log information on the memory of the sensor module, the process may communicate a current status of the log information stored in the memory of the sensor module. Alternatively or additionally, the analyzer may itself maintain a cache of log information during off-line periods such that the cached information is transmitted to the remote host system when the analyzer is on-line again.

Fig. 6 illustrates a flow diagram of an embodiment of a process for performing a quality-control measurement by an analyzer for analyzing biological samples, e.g. by the analyzer of FIG. 1.

In step S61, the process performs a quality-control measurement. For example, the analyzer may perform one or more quality control measurements periodically, after a certain number of analyzed samples, responsive to a user input, upon start-up of the analyzer, and/or the like. These measurements may involve performing a measurement of a known sample, e.g. a quality control liquid, for which the correct measurement result(s) is/are known. Accordingly, such measurements allow the analyzer to determine whether the actual measurement results performed on such a quality control sample are within acceptable deviations from the known target result.

In the present embodiment, at step S62, the process determines based on the result of the quality-control measurement whether one or more of the operational parameters should be modified. This determination may be based on a set of predetermined rules or on a more complex algorithm. For example, one of such predetermined rules may define one or more safety margins around a target measurement result for a certain quality control sample. Depending whether the actual measurement results falls within or outside these margins, the process may modify (e.g. increase or reduce) the maximum remaining number of samples associated with the present sensor module. Accordingly, the actual operational lifetime of the sensor module may be adjusted responsive to the module's performance in the quality control measurements. To this end, the determination step may, in some embodiments, not only take the current quality-control measurements into account but also previous quality control measurements, e.g. so as to determine a trend.

It will be appreciated that the above determination may be performed by the analyzer and/or by the remote host system. In the latter case, the results of the quality control measurements may be communicated to the remote host system which may then perform the determination of possibly modified operational parameters and return the thus modified operational parameters to the analyzer. In any event, in some embodiments the modification may in some embodiments be subject to an approval by an authorized operator.

In subsequent step S63, the process writes the modified operational parameters to the memory of the sensor module and uses the modified operational parameters for subsequent measurements.

If the analyzer is currently online, i.e. is able to communicate with the remote host system, the process may optionally proceed at step S64 and send the modified operational parameters to the remote host system, e.g. as described in connection with the log information of the process of FIG. 5.

Embodiments of the method described herein can be implemented by means of hardware comprising several distinct elements, and/or at least in part by means of a suitably programmed microprocessor.

In the claims enumerating several means, several of these means can be embodied by one and the same element, component or item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, elements, steps or components but does not preclude the presence or addition of one or more other features, elements, steps, components or groups thereof.

## Claims

1. A method of operating an apparatus (100) for analyzing biological samples, the apparatus comprising:
- a receptacle (110) for receiving a replaceable sensor module (200), the sensor module comprising one or more sensors (211) and a writable memory (220);
- a measurement unit (210) configured to bring a biological sample into operational interaction with at least a first sensor of the one or more sensors (211) of a sensor module (200) received by said receptacle (110), to obtain a measurement result responsive to the interaction between at least the first sensor and the biological sample and to output the obtained measurement result;
- a control unit (120) configured to control operation of the measurement unit (210);
- a data exchange interface (130) configured to receive data from the writable memory (220) and to forward data to the sensor module (200) for storage in the writable memory; and
- a communications interface (150) for communicating with a remote host system (300);
wherein the method comprises performing the following steps by the control unit:
a) receiving, from the remote host system (300) via said communications interface (150), one or more operational parameters associated with the sensor module (200);
b) causing writing, via said data exchange interface (130), the received one or more operational parameters to the writable memory (220); and
c) controlling operation of the measurement unit (210) in accordance with the received one or more operational parameters.

2. A method according to claim 1; wherein causing writing the received one or more operational parameters to the writable memory (220) comprises causing writing a time stamp to the writable memory, the time stamp being associated with the one or more operational parameters.

3. A method according to any one of the preceding claims; wherein receiving the one or more operational parameters comprises:
- receiving, via the data exchange interface (130), a module identifier from the writable memory of the sensor module (200) received by the receptacle (110);
- communicating, via the communications interface (150), the read module identifier to the remote host system (300);
- receiving, from the remote host system (300) via the communications interface (150), one or more operational parameters associated with the communicated module identifier.

4. A method according to claim 3; wherein communicating the read module identifier further comprises communicating a time stamp indicative of a time of reading the module identifier from the writable memory (220).

5. A method according to any one of the preceding claims; wherein receiving the one or more operational parameters comprises:
- communicating, via the communications interface (150), an analyzer identifier to the remote host system (300), the analyzer identifier identifying the apparatus for analyzing biological samples;
- receiving, from the remote host system (300) via the communications interface (150), one or more operational parameters associated with the communicated analyzer identifier.

6. A method according to any one of the preceding claims; comprising performing the following steps by the control unit:
- upon start-up of the apparatus (100), receiving information, via the data exchange interface (130), from the writable memory (220) of a sensor module (200) received by the receptacle (110);
- determining, based on the received information, whether or not the sensor module (200) has previously been used;
- responsive to determining that the sensor module (200) has not previously been used, performing steps a) through c); otherwise controlling operation of the measurement unit (210) in accordance with one or more previously stored operational parameters read from the writable memory (220) via the data exchange interface (130).

7. A method according to any one of the preceding claims; comprising performing the following steps by the control unit:
- upon start-up of the apparatus (100), determining whether or not communication with the remote host system (300) can be established via the communications interface (150);
- responsive to determining that communication with the remote host system (300) can be established, performing steps a) through c); otherwise controlling operation of the measurement unit (210) in accordance with one or more previously stored operational parameters read from the writable memory (220) via the data exchange interface (130).

8. A method according to any one of the preceding claims; comprising performing the following steps by the control unit:
- controlling the measurement unit (210) to perform one or more quality control measurements;
- responsive to a result of the one or more quality control measurements, obtaining one or more modified operational parameters associated with the sensor module (200) received by the receptacle (110);
- causing writing, via said data exchange interface (130), the one or more modified operational parameters to the writable memory (220); and
- controlling operation of the measurement unit (210) in accordance with the one or more modified operational parameters.

9. A method according to any one of the preceding claims; comprising causing, by the control unit (120) responsive to operation of the measurement unit (210), writing usage data to the writable memory (220) via the data exchange interface (130), the usage data being indicative of one or more usage parameters.

10. A method according to any one of the preceding claims; wherein the one or more operational parameters associated with the sensor module (200) include one or more parameters chosen from:
- a lock/unlock flag indicative of whether the control unit (120) is to allow measurements to be performed with the sensor module (200);
- one or more feature lock/unlock flags indicative of whether the control unit (120) is to lock or unlock one or more selected features of the apparatus (100) with the sensor module (200) received by the receptacle (110);
- one or more usage restriction parameters restricting usage of the sensor module (200) to one or more locations and/or operators and/or health care facilities;
- one or more start-up parameters indicative of a required start-up configuration of the apparatus (100) with the sensor module (200) received by the receptacle (110);
- one or more measurement parameters indicative of a required measurement configuration of the apparatus (100) with the sensor module (200) received by the receptacle (110).

11. A method according to any one of the preceding claims; comprising:
- maintaining, in a data storage of the apparatus (100), a local cache of one or more cached operational parameters;
- responsive to determining that the sensor module (200) has not previously been used and that communication with the remote host system (300) cannot be established, causing writing, via said data exchange interface (130), the cached operational parameters to the writable memory (220); and controlling operation of the measurement unit (210) in accordance with the cached one or more operational parameters.

12. A method according to claim 11; comprising, responsive to determining that communication with the remote host system (300) can again be established:
- receiving, from the remote host system (300) via said communications interface (150), one or more updated operational parameters associated with the sensor module (200);
- causing writing, via said data exchange interface (130), the received one or more updated operational parameters to the writable memory (220); and
- controlling operation of the measurement unit (210) in accordance with the received one or more updated operational parameters.

13. An apparatus (100) for analyzing biological samples, the apparatus comprising:
- a receptacle (110) for receiving a replaceable sensor module (200), the sensor module comprising one or more sensors (211) and a writable memory (220);
- a measurement unit (210) configured to bring a biological sample into operational interaction with at least a first sensor of the one or more sensors (211) of a sensor module (200) received by said receptacle (110), to obtain a measurement result responsive to the interaction between at least the first sensor and the biological sample and to output the obtained measurement result;
- a control unit (120) configured to control operation of the measurement unit (210);
- a data exchange interface (130) configured to receive data from the writable memory (220) and to forward data to the sensor module (200) for storage in the writable memory; and
- a communications interface (150) for communicating with a remote host system (300);
wherein the control unit (120) is configured to perform the steps of the method defined in any one of the preceding claims.

14. A system for analyzing biological samples, the system comprising:
- an apparatus (100) as defined in claim 13;
- a replaceable sensor module (200) comprising one or more sensors (211) and a writable memory (220); and
- a remote host system (300) configured to send one or more operational parameters associated with the sensor module (200) to the apparatus (100).

## Patentansprüche

1. Verfahren zum Betreiben einer Vorrichtung (100) zum Analysieren biologischer Proben, wobei die Vorrichtung umfasst:
- eine Aufnahme (110) zur Aufnahme eines austauschbaren Sensormoduls (200), wobei das Sensormodul einen oder mehrere Sensoren (211) und einen beschreibbaren Speicher (220) umfasst;
- eine Messeinheit (210), die so konfiguriert ist, dass sie eine biologische Probe in betriebliche Wechselwirkung mit mindestens einem ersten Sensor des einen oder der mehreren Sensoren (211) eines Sensormoduls (200) bringt, das von der Aufnahme (110) aufgenommen wird, um ein Messergebnis zu erhalten, das auf die Wechselwirkung zwischen mindestens dem ersten Sensor und der biologischen Probe reagiert, und um das erhaltene Messergebnis auszugeben;
- eine Steuereinheit (120), die so konfiguriert ist, dass sie den Betrieb der Messeinheit (210) kontrolliert;
- eine Datenaustauschschnittstelle (130), die so konfiguriert ist, dass sie Daten von dem beschreibbaren Speicher (220) empfängt und Daten an das Sensormodul (200) zur Speicherung in dem beschreibbaren Speicher weiterleitet; und
- eine Kommunikationsschnittstelle (150) zum Kommunizieren mit einem entfernten Hostsystem (300) ;
wobei das Verfahren die Durchführung der folgenden Schritte durch die Steuereinheit umfasst:
a) Empfangen eines oder mehrerer Betriebsparameter, die dem Sensormodul (200) zugeordnet sind, von dem entfernten Hostsystem (300) über die Kommunikationsschnittstelle (150);
b) Veranlassen des Schreibens des bzw. der empfangenen einen oder der mehreren Betriebsparameter in den beschreibbaren Speicher (220) über die Datenaustauschschnittstelle (130); und
c) Steuern des Betriebs der Messeinheit (210) in Übereinstimmung mit den empfangenen einen oder mehreren Betriebsparametern.

2. Verfahren nach Anspruch 1, wobei das Veranlassen des Schreibens des oder der empfangenen Betriebsparameter in den beschreibbaren Speicher (220) das Veranlassen des Schreibens eines Zeitstempels in den beschreibbaren Speicher umfasst, wobei der Zeitstempel mit dem einen oder den mehreren Betriebsparametern verbunden ist.

3. Verfahren nach einem der vorhergehenden Ansprüche, wobei das Empfangen des einen oder der mehreren Betriebsparameter umfasst:
- Empfangen einer Modulkennung aus dem beschreibbaren Speicher des Sensormoduls (200), das von der Aufnahme (110) empfangen wurde, über die Datenaustauschschnittstelle (130);
- Übermitteln der gelesenen Modulkennung an das entfernte Hostsystem (300) über die Kommunikationsschnittstelle (150);
- Empfangen eines oder mehrerer Betriebsparameter, die mit der übermittelten Modulkennung verbunden sind, von dem entfernten Hostsystem (300) über die Kommunikationsschnittstelle (150).

4. Verfahren nach Anspruch 3, wobei die Übermittlung der gelesenen Modulkennung ferner die Übermittlung eines Zeitstempels umfasst, der den Zeitpunkt des Lesens der Modulkennung aus dem beschreibbaren Speicher (220) angibt.

5. Verfahren nach einem der vorhergehenden Ansprüche, wobei das Empfangen des einen oder der mehreren Betriebsparameter umfasst:
- Übermitteln einer Analyzer-Kennung über die Kommunikationsschnittstelle (150) an das entfernte Hostsystem (300), wobei die Analyzer-Kennung das Gerät zum Analysieren biologischer Proben identifiziert;
- Empfangen über die Kommunikationsschnittstelle (150) einen oder mehrere Betriebsparameter, die mit der übermittelten Analysator-Kennung verbunden sind, von dem entfernten Hostsystem (300).

6. Verfahren nach einem der vorhergehenden Ansprüche, bei dem die Steuereinheit die folgenden Schritte durchführt:
- Empfangen von Informationen über die Datenänderungsschnittstelle (130) aus dem beschreibbaren Speicher (220) eines Sensormoduls (200), das von der Aufnahme (110) empfangen wurde, beim Starten der Vorrichtung (100);
- Bestimmen, basierend auf den empfangenen Informationen, ob das Sensormodul (200) zuvor verwendet wurde oder nicht;
- als Reaktion auf die Feststellung, dass das Sensormodul (200) zuvor nicht verwendet wurde, Ausführen der Schritte a) bis c); ansonsten Steuern des Betriebs der Messeinheit (210) in Übereinstimmung mit einem oder mehreren zuvor gespeicherten Betriebsparametern, die über die Datenaustauschschnittstelle (130) aus dem beschreibbaren Speicher (220) gelesen wurden.

7. Verfahren nach einem der vorhergehenden Ansprüche, umfassend die Ausführung der folgenden Schritte durch die Steuereinheit:
- beim Starten der Vorrichtung (100) Feststellen, ob eine Kommunikation mit dem entfernten Hostsystem (300) über die Kommunikationsschnittstelle (150) hergestellt werden kann oder nicht;
- als Reaktion auf die Feststellung, dass die Kommunikation mit dem entfernten Hostsystem (300) hergestellt werden kann, Ausführen der Schritte a) bis c); andernfalls Steuern des Betriebs der Messeinheit (210) in Übereinstimmung mit einem oder mehreren zuvor gespeicherten Betriebsparametern, die aus dem beschreibbaren Speicher (220) über die Datenaustauschschnittstelle ausgelesen wurden.

8. Verfahren nach einem der vorhergehenden Ansprüche, umfassend die Durchführung der folgenden Schritte durch die Steuereinheit:
- Steuern der Messeinheit (210), um eine oder mehrere Qualitätskontrollmessungen durchzuführen;
- als Reaktion auf ein Ergebnis der einen oder mehreren Qualitätskontrollmessungen, Erhalten eines oder mehrerer modifizierter Betriebsparameter, die dem Sensormodul (200) zugeordnet sind, das von der Aufnahme (110) empfangen wird;
- Veranlassen des Schreibens des einen oder der mehreren modifizierten Betriebsparameter in den beschreibbaren Speicher (220) über die Datenaustauschschnittstelle (130); und
- Steuern des Betriebs der Messeinheit (210) in Übereinstimmung mit dem einen oder den mehreren modifizierten Betriebsparametern.

9. Verfahren nach einem der vorhergehenden Ansprüche, umfassend das Veranlassen des Schreibens von Nutzungsdaten in den beschreibbaren Speicher (220) über die Datenaustauschschnittstelle (130) durch die Steuereinheit (120) als Reaktion auf den Betrieb der Messeinheit (210), wobei die Nutzungsdaten einen oder mehrere Nutzungsparameter anzeigen.

10. Verfahren nach einem der vorhergehenden Ansprüche, wobei der eine oder die mehreren Betriebsparameter, die dem Sensormodul (200) zugeordnet sind, einen oder mehrere Parameter umfassen, die ausgewählt sind aus:
- einem Sperr-/Entsperr-Flag, das anzeigt, ob die Steuereinheit (120) die Durchführung von Messungen mit dem Sensormodul (200) zulassen soll;
- ein oder mehrere Merkmals-Sperr-/Entsperr-Flags, die anzeigen, ob die Steuereinheit (120) ein oder mehrere ausgewählte Merkmale der Vorrichtung (100) mit dem Sensormodul (200), das von der Aufnahme (110) empfangen wird, sperren oder entsperren soll;
- einen oder mehrere Verwendungsbeschränkungsparameter, die die Verwendung des Sensormoduls (200) auf einen oder mehrere Orte und/oder Bediener und/oder Gesundheitseinrichtungen beschränken;
- einen oder mehrere Startparameter, die eine erforderliche Startkonfiguration der Vorrichtung (100) mit dem von der Aufnahme (110) empfangenen Sensormodul (200) angeben;
- einen oder mehrere Messparameter, die eine erforderliche Messkonfiguration der Vorrichtung (100) mit dem von der Aufnahme (110) aufgenommenen Sensormodul (200) anzeigt.

11. Verfahren nach einem der vorangehenden Ansprüche, umfassend:
- Verwalten eines lokalen Caches mit einem oder mehreren zwischengespeicherten Betriebsparametern in einem Datenspeicher der Vorrichtung (100);
- als Reaktion auf die Feststellung, dass das Sensormodul (200) zuvor nicht verwendet wurde und dass keine Kommunikation mit dem entfernten Hostsystem (300) hergestellt werden kann, Veranlassen des Schreibens der zwischengespeicherten Betriebsparameter über die Datenaustauschschnittstelle (130) in den beschreibbaren Speicher (220); und Steuern des Betriebs der Messeinheit (210) gemäß dem einen oder den mehreren zwischengespeicherten Betriebsparametern.

12. Verfahren nach Anspruch 11, das als Reaktion auf die Feststellung, dass die Kommunikation mit dem entfernten Hostsystem (300) wieder hergestellt werden kann, Folgendes umfasst:
- Empfangen eines oder mehrerer aktualisierter Betriebsparameter, die dem Sensormodul (200) zugeordnet sind, von dem entfernten Hostsystem (300) über die Kommunikationsschnittstelle (150);
- Veranlassen des Schreibens des empfangenen einen oder der mehreren aktualisierten Betriebsparameter in den beschreibbaren Speicher (220) über die Datenaustauschschnittstelle (130); und
- Steuern des Betriebs der Messeinheit (210) in Übereinstimmung mit dem einen oder den mehreren empfangenen aktualisierten Betriebsparametern.

13. Vorrichtung (100) zum Analysieren biologischer Proben, wobei die Vorrichtung umfasst:
- eine Aufnahme (110) zum Aufnehmen eines austauschbaren Sensormoduls (200), wobei das Sensormodul einen oder mehrere Sensoren (211) und einen beschreibbaren Speicher (220) umfasst;
- eine Messeinheit (210), die so konfiguriert ist, dass sie eine biologische Probe mit mindestens einem ersten Sensor des einen oder der mehreren Sensoren (211) eines von der Aufnahme (110) aufgenommenen Sensormoduls (200) in betriebliche Wechselwirkung bringt, um ein Messergebnis zu erhalten auf die Interaktion zwischen zumindest dem ersten Sensor und der biologischen Probe zu reagieren und das erhaltene Messergebnis auszugeben;
- eine Steuereinheit (120), die so konfiguriert ist, dass sie den Betrieb der Messeinheit (210) steuert;
- eine Datenaustauschschnittstelle (130), die dazu konfiguriert ist, Daten vom beschreibbaren Speicher (220) zu empfangen und Daten an das Sensormodul (200) zur Speicherung im beschreibbaren Speicher weiterzuleiten; und
- eine Kommunikationsschnittstelle (150) zum Kommunizieren mit einem entfernten Hostsystem (300);
wobei die Steuereinheit (120) so konfiguriert ist, dass sie die Schritte des in einem der vorhergehenden Ansprüche definierten Verfahrens ausführt.

14. System zum Analysieren biologischer Proben, wobei das System umfasst
- eine Vorrichtung (100) wie in Anspruch 13 definiert;
- ein austauschbares Sensormodul (200) mit einem oder mehreren Sensoren (211) und einem beschreibbaren Speicher (220); und
- ein entferntes Hostsystem (300), das so konfiguriert ist, dass es einen oder mehrere mit dem Sensormodul (200) verknüpfte Betriebsparameter an die Vorrichtung (100) sendet.

## Revendications

1. Procédé de fonctionnement d'un appareil (100) pour analyser des échantillons biologiques, l'appareil comprenant :
- un réceptacle (110) pour recevoir un module de capteur remplaçable (200), le module capteur comprenant un ou plusieurs capteurs (211) et une mémoire inscriptible (220);
- une unité de mesure (210) configurée pour amener un échantillon biologique en interaction opérationnelle avec au moins un premier capteur parmi le ou les capteurs (211) d'un module de capteur (200) reçu par ledit réceptacle (110), pour obtenir un résultat de mesure sensible à l'interaction entre au moins le premier capteur et l'échantillon biologique et pour délivrer le résultat de mesure obtenu ;
- une unité de commande (120) configurée pour commander le fonctionnement de l'unité de mesure (210);
- une interface d'échange de données (130) configurée pour recevoir des données de la mémoire inscriptible (220) et pour transmettre des données au module de capteur (200) en vue d'une mémorisation dans la mémoire inscriptible ; et
- une interface de communication (150) pour communiquer avec un système hôte distant (300);
dans lequel le procédé comprend l'exécution des étapes suivantes par l'unité de commande :
a) recevoir, du système hôte distant (300) via ladite interface de communication (150), un ou plusieurs paramètres opérationnels associés au module de capteur (200) ;
b) provoquer l'écriture, via ladite interface d'échange de données (130), du ou des paramètres opérationnels reçus dans la mémoire inscriptible (220) ; et
c) commander le fonctionnement de l'unité de mesure (210) conformément au ou aux paramètres opérationnels reçus.

2. Procédé selon la revendication 1 ; dans lequel provoquer l'écriture du ou des paramètres opérationnels reçus dans la mémoire inscriptible (220) comprend le fait de provoquer l'écriture d'un pointeur temporel dans la mémoire inscriptible, le pointeur temporel étant associé au ou aux paramètres opérationnels.

3. Procédé selon une quelconque des revendications précédentes ; dans lequel la réception d'un ou plusieurs paramètres opérationnels comprend de :
- recevoir, via l'interface d'échange de données (130), un identifiant de module issu de la mémoire inscriptible du module de capteur (200) reçu par le réceptacle (110);
- communiquer, via l'interface de communication (150), le module de lecture identifiant du système hôte distant (300) ;
- recevoir, du système hôte distant (300) via l'interface de communication (150), un ou plusieurs paramètres opérationnels associés à l'identifiant de module communiqué.

4. Procédé selon la revendication 3 ; dans lequel la communication de l'identifiant de module de lecture comprend en outre la communication d'un pointeur temporel indiquant l'heure de lecture de l'identifiant de module à partir de la mémoire inscriptible (220).

5. Procédé selon une quelconque des revendications précédentes ; dans lequel la réception d'un ou plusieurs paramètres opérationnels comprend de :
- communiquer, via l'interface de communication (150), un identifiant d'analyseur au système hôte distant (300), l'identifiant d'analyseur identifiant l'appareil d'analyse d'échantillons biologiques ;
- recevoir, du système hôte distant (300) via l'interface de communication (150), un ou plusieurs paramètres opérationnels associés à l'identifiant d'analyseur communiqué.

6. Procédé selon une quelconque des revendications précédentes ; comprenant l'exécution des étapes suivantes par l'unité de commande :
- au démarrage de l'appareil (100), recevoir des informations, via l'interface d'échange de données (130), depuis la mémoire inscriptible (220) d'un module de capteur (200) reçu par le réceptacle (110) ;
- déterminer, sur la base des informations reçues, si le module de capteur (200) a déjà été utilisé ou non,
- en réponse à la détermination du fait que le module de capteur (200) n'a pas été utilisé précédemment, exécuter les étapes a) à c) ; sinon commander le fonctionnement de l'unité de mesure (210) conformément à un ou plusieurs paramètres opérationnels préalablement mémorisés et lus dans la mémoire inscriptible (220) via l'interface d'échange de données (130).

7. Procédé selon une quelconque des revendications précédentes ; comprenant l'exécution des étapes suivantes par l'unité de commande :
- lors du démarrage de l'appareil (100), déterminer si une communication avec le système hôte distant (300) peut être établie via l'interface de communication (150) ;
- en réponse à la détermination que cette communication avec le système hôte distant (300) peut être établie, exécuter les étapes a) à c); sinon, commander le fonctionnement de l'unité de mesure (210) conformément à un ou plusieurs paramètres opérationnels précédemment mémorisés, lus dans la mémoire inscriptible (220) via l'interface d'échange de données (130).

8. Procédé selon une quelconque des revendications précédentes ; comprenant l'exécution des étapes suivantes par l'unité de commande :
- commander l'unité de mesure (210) pour exécuter une ou plusieurs mesures de contrôle de qualité ;
- en réponse à un résultat de la ou des mesures de contrôle de qualité, obtenir un ou plusieurs paramètres opérationnels modifiés associés au module de capteur (200) reçu par le réceptacle (110) ;
- provoquer l'écriture, via ladite interface d'échange de données (130) du ou des paramètres opérationnels modifiés dans la mémoire inscriptible (220) ; et
- commander le fonctionnement de l'unité de mesure (210) conformément à un ou plusieurs paramètres opérationnels modifiés.

9. Procédé selon une quelconque des revendications précédentes ; comprenant de provoquer, par l'unité de commande (120) en réponse au fonctionnement de l'unité de mesure (210), l'écriture des données d'utilisation dans la mémoire inscriptible (220) via l'interface d'échange de données (130), les données d'utilisation étant indicatives d'un ou plusieurs paramètres d'utilisation.

10. Procédé selon une quelconque des revendications précédentes ; dans lequel le ou les paramètres opérationnels associés au module de capteur (200) comprennent un ou plusieurs paramètres choisis parmi :
- un fanion de verrouillage/déverrouillage indiquant si l'unité de commande (120) doit autoriser l'exécution de mesures avec le module de capteur (200) ;
- un ou plusieurs fanions de verrouillage/déverrouillage de fonctionnalité indiquant si l'unité de commande (120) doit verrouiller ou déverrouiller une ou plusieurs fonctionnalités sélectionnées de l'appareil (100) avec le module de capteur (200) reçu par le réceptacle (110) ;
- un ou plusieurs paramètres de restriction d'utilisation limitant l'utilisation du module de capteur (200) à un ou plusieurs emplacements et/ou opérateurs et/ou installations de soins de santé ;
- un ou plusieurs paramètres de démarrage indicatifs d'une configuration de démarrage requise de l'appareil (100) avec le module de capteur (200) reçu par le réceptacle (110) ;
- un ou plusieurs paramètres de mesure indicatifs d'une configuration de mesure requise de l'appareil (100) avec le module capteur (200) reçu par le réceptacle (110).

11. Procédé selon une quelconque des revendications précédentes ; comprenant de :
- maintenir, dans un stockage de données de l'appareil (100), une mémoire cache locale d'un ou plusieurs paramètres opérationnels supplémentaires mis en mémoire cache ;
- en réponse à la détermination du fait que le module de capteur (200) n'a pas été utilisé auparavant et que la communication avec le système hôte distant (300) ne peut pas être établie, provoquer l'écriture, via ladite interface d'échange de données (130), des paramètres opérationnels mis en mémoire cache sur la inscriptible mémoire (220); et commander le fonctionnement de l'unité de mesure (210) conformément au ou aux paramètres opérationnels mis en mémoire cache.

12. Procédé selon la revendication 11 ; comprenant, en réponse à la détermination que la communication avec le système hôte distant (300) peut à nouveau être établie de :
- recevoir, du système hôte distant (300) via ladite interface de communication (150), un ou plusieurs paramètres opérationnels mis à jour associés au module de capteur (200) ;
- provoquer l'écriture, via ladite interface d'échange de données (130), du ou des paramètres opérationnels mis à jour reçus dans la mémoire inscriptible (220) ; et
- commander le fonctionnement de l'unité de mesure (210) conformément au ou aux paramètres opérationnels mis à jour reçus.

13. Appareil (100) pour analyser des échantillons biologiques, l'appareil comprenant :
- un réceptacle (110) pour recevoir un module de capteur remplaçable (200), le module de capteur comprenant un ou plusieurs capteurs (211) et une mémoire inscriptible (220);
- une unité de mesure (210) configurée pour amener un échantillon biologique en interaction opérationnelle avec au moins un premier capteur parmi le ou les capteurs (211) d'un module de capteur (200) reçu par ledit réceptacle (110), pour obtenir un résultat de mesure en réponse à l'interaction entre au moins le premier capteur et l'échantillon biologique et permettant de délivrer le résultat de mesure obtenu;
- une unité de commande (120) configurée pour commander le fonctionnement de l'unité de mesure (210);
- une interface d'échange de données (130) configurée pour recevoir des données de la mémoire inscriptible (220) et pour transmettre des données au module de capteur (200) en vue d'une mémorisation dans la mémoire inscriptible ; et
- une interface de communication (150) pour communiquer avec un système hôte distant (300) ;
dans lequel l'unité de commande (120) est configurée pour exécuter les étapes du procédé défini dans une quelconque des revendications précédentes.

14. Système d'analyse d'échantillons biologiques, le système comprenant :
- un appareil (100) tel que défini dans la revendication 13 ;
- un module de capteur remplaçable (200) comprenant un ou plusieurs capteurs (211) et une mémoire inscriptible (220) ; et
- un système hôte distant (300) configuré pour envoyer un ou plusieurs paramètres opérationnels associés au module de capteur (200) à l'appareil (100) .
